(19) Europäisches Patentamt
European Patent Office
Office européen des brevets

(11) **EP 2 738 267 B1**

## (12) EUROPEAN PATENT SPECIFICATION

(45) Date of publication and mention
of the grant of the patent:
**10.02.2016 Bulletin 2016/06**

(21) Application number: **12464030.1**

(22) Date of filing: **04.12.2012**

(51) Int Cl.:
***C12R 1/64*** (2006.01)

(54) **Strain of Pseudoxanthomonas mexicana and controlled release composition which contain said strain**

Stamm von Pseudoxanthomonas mexicana und Zusammensetzung mit kontrollierter Freisetzung, die der Stamm enthalten

Souche de Pseudoxanthomonas mexicana et composition à libération contrôlée qui contient ladite souche

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.12.2012 RO 201200931**

(43) Date of publication of application:
**04.06.2014 Bulletin 2014/23**

(83) **Declaration under Rule 32(1) EPC (expert
solution)**

(73) Proprietor: **SC Euro Envirotech SRL
100481 Ploiesti, Jud. Prahova (RO)**

(72) Inventors:
• **Niculae, Gheorghe
100481 Ploiesti
Jud. Prahova (RO)**
• **Oancea, Florin
062082 Bucharest (RO)**
• **Rusen, Rodica
107070 com. Blejoi
Jud. Prahova (RO)**
• **Doni, Mihaela
031747 Bucharest (RO)**
• **Raut, Iuliana
032768 Bucharest (RO)**
• **Calin, Mariana
060962 Bucharest (RO)**
• **Jecu, Maria-Luiza
060144 Bucharest (RO)**

(74) Representative: **Teodorescu, Mihaela
Mihaela Teodorescu & Partners
Intellectual Property Office S.R.L.
P.O.Box 53-202
51 Viorele St. bl.37entrance 2 ap.63 sector 4
040425 Bucharest (RO)**

(56) References cited:
**JP-A- 2008 289 445**

• **S. THIERRY: "Pseudoxanthomonas mexicana sp.
nov. and Pseudoxanthomonas japonensis sp.
nov., isolated from diverse environments, and
emended descriptions of the genus
Pseudoxanthomonas Finkmann et al. 2000 and of
its type species", INTERNATIONAL JOURNAL OF
SYSTEMATIC AND EVOLUTIONARY
MICROBIOLOGY, vol. 54, no. 6, 1 November 2004
(2004-11-01), pages 2245-2255, XP055085173,
ISSN: 1466-5026, DOI: 10.1099/ijs.0.02810-0**

**Description**

The present invention refer to a strain of *Pseudoxanthomonas mexicana* which has the ability to degrade aromatic and aliphatic hydrocarbons, stimulates plant growth and is antagonistic to a number of phytopathogen agents, and to a controlled-release composition containing this strain, intended to be used in the processes of bio- and phyto/rhizoremediation of soils contaminated with petroleum products, including those contaminated with polycyclic aromatic hydrocarbons, potentially carcinogenic.

There are known a number of strains of microorganisms, particularly bacteria from the Proteobacteria phylum, which degrades the aromatic and aliphatic hydrocarbons, as well as their derivatives, from contaminated soils. US Patent 5543317 refers to a strain of *Pseudomonas cepacia* (later re-classified as species as *Burkholderia cepacia*) $PR_{123}$, originally called G4 5223 Phe, stored under number NRRL B-18811, which has the ability to degrade hazardous chemicals from the category of chlorinated derivatives of alkanes, trichloroethylene, 1,1-dichloroethane, cis-1,2-dichloroethylene, trans-1,2-dichloroethylene, and aromatic hydrocarbons, p-toluene, phenol, o-cresol, m-cresol, o-xylene and benzene. Patent Application KR20030066948 protects a strain of *Burkholderia cepacia* 1A-12, deposited under number KCTC 10163BP, with high capacity to degrade aromatic hydrocarbons, and a process of bioremediation that involves spraying on the contaminated area with the said strain and with yeast extract. Patent application CN102250788 describe a strain of *Stenotrophomonas maltophilia*, deposited under number 4839 CGMCC, with high capacity to use methyl-benzene / toluene as the sole source of carbon and energy, and to degrade and tolerate toluene.

These two species of proteobacteria mentioned above, *Burkholderia cepacia* and *Stenotrophomonas maltophilia*, are species with an high eco-biotechnological versatility, which degrade organic polluting compounds, including hydrocarbons and their derivatives, have antagonistic activity against phytopathogens agents and for cultivated plant bio-stimulation, bio-synthesize useful compounds, including antibiotics - see for example reviews of Mahenthiralingam et al., 2008, J. Appl. Microbiol. 104: 1539-1551 for *B. cepacia* or Ryan et al., Nat. Rev. Microbiol 7: 514-525 for *S. maltophilia*. These two species are also opportunistic human pathogens, showing a high resistance to antibiotics, which produce serious intra-hospital infections (see for example here the reviews of Govan and Deretic, 1996. Microbiol. Rev. 60:539-574 for *B. cepacia* or Looney, 2009, Lancet Infect. Dis 9: 312-323 for *S. maltophilia*). For this reason, their use in applications in the processes of bio- and phyto/rhizoremediation involve risks to human health,

Patent JP3618785 discloses a process through which the bioremediation is done by cells lysates of *Burkholderia* (*Pseudomonas*) *cepacia*, strain KK01, deposited as a FERM-BP-4235. Thus are avoided the subsequent risks to human health, but such a process involves the use of a very large amounts of lysate of cells, which shall provide the amount of enzymes needed for decontamination, and no longer offers the advantage of using a (relatively) small quantity of the micro-organism strain, with high efficiency in the biodegradation of organic pollutants, as an inoculant which further colonize the ecological niche, including here the plant rhizosphere. Cells lysate is not useful in phyto(rhizo)remediation process, wherein organic compounds degradation is done through a combined action of plant roots (and of exudate compounds from the roots) and of the strains of micro-organisms which develop in the rhizosphere, co-metabolizing plants exudates and organic pollutants, and supporting the development of plants (for the description of the processes and mechanisms involved, see for example the reviews of Kuiper et al. 2004. Mol. Plant Microbe Interact. 17:6-15; Lugtenberg and Kamilova, 2009. Annu. Rev. Microbiol. 63:541-556).

Thus is necessary to identify strains of proteobacteria with an eco-biotechnological versatility similar to *B. cepacia* and *S. maltophilia*, useful both for direct bio-remediation and for remediation together with plant roots, stimulated and protects against different phytopathogens by these proteobacteria. Patent RU2406758 claims the strain *Sinorhizobium meliloti* P221, deposited under number B-9442 on Russian National Collection of Industrial Microorganisms, VKPM, which degrade polycyclic aromatic hydrocarbons, being useful both for bioremediation and phyto(rhizo)remediation. For this strain were not yet described characteristics of antagonism for the phytopathogen agents, so this strain only stimulate the cultivated plants, without protecting them against the attack of phytopathogen agents.

The technical problem that aims to solve the invention is to disclose a strain, isolated from natural habitats, which, in the same time, present the following characteristics: degradation of aliphatic and aromatic hydrocarbons, stimulation of plant growth and antagonism for a certain number of phytopathogen agents. For such strains is required also a controlled release composition, which should: ensure an optimum water activity for long-term survival of metabolically inactive forms of proteobacteria, promote the colonization of rhizosphere with the micro-organism through an extended and gradual release, and protect the proteobacteria from the toxic action of hydrocarbons during the critical phase of reactivation of metabolically inactive forms.

This invention refer to a strain of *Pseudoxanthomonas mexicana*, deposited with the deposit number NCAIM (P) B 001414, at National Collection of Agricultural and Industrial Microorganisms, Corvinus University of Budapest. Strain *Pseudoxanthomonas mexicana* P32 was selected from more than 120 isolated from samples of soil contaminated with hydrocarbons, and grown on a minimal medium supplemented with phenanthrene as the sole carbon source, presenting superior characteristics of:

✔ degradation of aliphatic and aromatic hydrocarbons in various environments, including soil, and including in combination with alfalfa roots;

✔ biofilms production *in vitro* and *in situ;*

✔ biosynthesis of volatile compounds with plant growth stimulation action and of compounds which solubilize phosphorus from its insoluble forms, organic and inorganic;

✔ stimulation of alfalfa seed germination and alfalfa seedling development, under controlled conditions;

✔in vitro antagonism towards phytopathogenic fungi, *Rhizoctonia solani, Fusarium graminearum, Pythium ultimum, Phytophthora megasperma* f. sp. *medicaginis, Sclerotinia sclerotiorum, Aphanomyces euteiches, Botrytis cinerea;*

✔In vivo protection of alfalfa seedling against phytopathogenic fungi of soil, *R. solani, F. graminearum, P. ultimum, P. megasperma* f. sp. *medicaginis, Aphanomyces euteiches*) which produce seedling damping-off;

✔ Innocuity on *Galleria mellonella* test.

The controlled-release composition based on strain *of P. mexicana* P32 consists of 76 parts oatmeal flour, 4.8 parts ethyl esters of fatty acids, 4 parts polyvinyl alcohol, 3.5 parts lecithin, 0.75 parts potassium soap, 0.45 parts glycerol, 0.4 parts of fats from rapeseed oil, the rest till 100 parts water, and min. $10^8$ cfu/g *P. mexicana* P32.

By the application of the invention are obtained the following advantages:

- possibility for bioremediation of soils contaminated with petroleum products, including carcinogenic hydrocarbons, by introducing of a suspension from the strain *P. mexicana* P32, or granules of controlled-release composition, into the soil;
- possibility for phytorhizoremediation of soils contaminated with petroleum products, by using in-furrow treatment during seed drilling, with a suspension of the strain *P. mexicana* P32 or granules of controlled-release composition;
- stimulation and protection of aifaifa crop, used together with the strain *P. mexicana* P32 for phytorhizoremediation, reducing the cost of crop maintenance and increasing the efficacy of the phytorhizoremediation process;
- ensure a long term survival of the metabolically inactive forms of proteobacteria *P. mexicana* P32 in the controlled release composition, due to an optimal water activities in substrate, resulted from humectant action of glycerol and water repellent action of the hydrophobic compounds;
- ensure an uniform and reproducible colonization of soil by P32 strain, when the controlled-release composition is used, the propagules being gradually released from the composition according to this invention, and protected from the toxic hydrocarbons action, during critical phase of reactivation of metabolically inactive forms, as a result of the prevalent accumulation of hydrocarbons in hydrophobic component of the composition.

Further the invention will be described in details in the following examples.

*Example 1.* Isolation of P32 strain was done from soil samples contaminated with petroleum products taken from Brazi, Ploiesti, Romania. For isolation it was used a minimal medium ($KH_2PO_4$ 1.0 g/l, $K_2HPO_4$ 1.0 g/l, NaCl 0.5 g/l, $NH_4NO_3$ 1.0 g/l, $MgSO_4$. 7 $H_2O$ 0.2 g/l, $CaCl_2$ 20 mg/l and $FeCl_3$. $6H_2O$ 5 mg/l), supplemented with phenanthrene as a sole source of carbon and energy. The chemicals used for medium isolation were from Sigma-Aldrich (St. Louis, MO, USA). pH of the medium was adjusted to 7.2 with 0.1 N NaOH, and the medium was sterilized by autoclavation at 121°C for 20 min.

Soil samples taken from the polluted areas with petroleum products were brought to a dimension of less than 0.2 mm by sieving and then inoculated on the minimal medium supplemented 50 mg/l phenanthrene. Samples were maintained at 32°C on an orbital shaker (Unimax 1010, Heidolph Instruments Schwabach, and Germany). After one week a part of this sample was used as inoculum for a fresh, minimal medium, supplemented with 75 mg/l phenanthrene, which was maintained for other seven days at 32°C for enrichment, on an aerated and agitated medium, on the orbital shaker. This procedure was repeated 10 times, with an increase of the content of phenanthrene in the medium with 25 mg/l each time, until reaching the concentration of 300 mg/l. From the last samples, taken from the minimal medium enriched with 300 mg/l phenanthrene, were made dilutions which were spread into 25 ml of minimal medium, agarized with 20 g/l agar, sterilized by autoclavation at 121°C for 20 min, and distributed in sterile Petri dishes, diameter 9 cm. After distribution of the minimal medium in Petri dishes and cooling it, on the surface of the agarized medium were spread 0.5 ml of 0.25% solution phenanthrene in acetone, corresponding to a final concentration of phenanthrene in the agarized medium of 50 mg/l. After evaporation of acetone under aseptic conditions, the medium was inoculated with dilutions of samples enriched in bacteria which use aromatic hydrocarbons as sole source of carbon and energy. Based on morphological characters were separated 121 isolates, which were maintained on minimal medium, agarized and supplemented with phenanthrene 50 mg/l, prepared as above.

From the 121 strains it was selected strain P32, based on the tests which will be detailed in the other examples. For taxonomic classification of P32 strain it was used a polyphasic approach, this strain being characterized in terms of morphological (table 1), biochemical and physiological (table 2) characteristics, of cellular fatty acid composition and of partial 16S rDNA sequence.

Tab. 1. Morphology of cell and colonies of *Pseudoxanthomonas mexicana* P32 on agarized LB medium, after growing for 24 hours.

| Morphological characters of typical *Mexican Pseudoxanthomonas* P32 | |
|---|---|
| Colonies | *Form:* circular |
| | *Surface appearance*: smooth, convex |
| | *Transparency*: translucent |
| | *Color*: pale yellowish to beige |
| Cells: | *Form*: rod-shaped |
| | *Dimensions*: 0.7 - 0.8 x 1.5-2.5 $\mu$m |
| | *Flagelar arrangement*: one polar flagellum |

Tab. 2. Physiological characteristics of strain P32.

| Biochemical test | P32 |
|---|---|
| Gram Reaction | - |
| Voges-Proskauer Reaction | + |
| Starch hydrolysis | + |
| Gelatin hydrolysis | + |
| Urease | - |
| Reduction $NO_3 \rightarrow_7 NO_2$ | - |
| Anaerobic growth | - |
| Oxidase | + |
| Catalase | + |
| Lecithinase | - |
| Carbon source: | |
| Gentiobiose | - |
| Galactose | - |
| Sucrose | + |
| Maltose | - |
| Glucose | - |
| Xylose | + |
| Mannose | + |
| Fructose | - |
| Trehalose | - |
| Sorbitol | + |
| Mannitol | + |
| Inositol | - |
| Glycerol | + |
| meso-Erythritol | + |
| D/L-Proline | + |
| Adipate | - |
| Phenylacetate | + |
| Malate | + |

*P. mexicana* P32 strain growth is possible in the presence of 0...40 g NaCl per 1 liter of medium, at 10 ...37°C and at a pH between 5.8 and 9.75, and is optimal at 30....37°C, pH 7...8, without NaCl.

Cellular fatty acid composition was determined using an automatic GC Sherlock Microbial Identification System (MIDI, Newark, USA) and standard MIDI method (Sasser, 1990, in Methods in Phytobacteriology, pp. 119-204. Z. James, K. Rudolph, and D. C. Sands, eds., Budapest: Akademiai Kiado). Predominant cellular fatty acids are, in order of decreasing abundance: 15: 0 iso, 16: 1 iso cis7, 16: 0 iso, 15: 0 iso 3-OH 14: 0 iso, corresponding to the profile of fatty acids cell-specific to species *Pseudoxanthomonas mexicana* (Rose et al., 2004. Int. J. System. Evol. Microbiol. 54:2245-2255).

The identification on the basis of 16S rDNA sequence was done by using a working protocol characterized by the following steps: obtaining of pure cultures - isolated colonies, inoculation techniques by loop streaking; extraction of bacterial DNA; gel electrophoresis for DNA detection; amplification of 16S rADN sequence through PCR technique and gel electrophoresis; purification of ribozomal DNA; enzymatic amplification of nucleic acids before sequencing; precipitation and drying of DNA coding for 16S RNA. Nucleotide sequencing was carried out with the method *Dye Terminator Cycle Sequencing* (Perkin Eimer, 1998), using an automated sequencer ABI PRISM 310 (Perkin Elmer). Sequences were analyzed using *CHROMAS* 2.33 (Technelysium Pty Ltd). Comparison of the sequences of 16S rDNA obtained, with existing sequences in the Gene Bank of NCBI (National Center for Biotechnology Information), was achieved with *BLAST* (Basic Local Alignment Search Tool). Partial sequence, of 527 base pairs, presented a 100% similarity with the sequence of the reference strain AMX 26B[T] / ATCC 700993[T] (Rose et al., 2004. Int. J. System. Evol. Microbiol. 54:2245-2255).

*Example 2.* It was tested the ability of different isolates to growth on a liquid minimal medium supplemented with different xenobiotic compounds as sole source of carbon and energy. It was used the same minimal medium as on Example 1. ($KH_2PO_4$ 1.0 g/l, $K_2HPO_4$ 1.0 g/l, NaCl 0.5 g/l, $NH_4NO_3$ 1.0 g/l, $MgSO_4$. 7 $H_2O$ 0.2 g/l, $CaCl_2$. $2H_2O$ 20 mg/l and $FeCl_3$. $6H_2O$ 5 mg/l), to which were added 1 g/l of various xenobiotic compounds, respectively acetone (ketones), n-hexane (alkanes), benzene, toluene and xylene (aromatic hydrocarbons), naphthalene, phenanthrene and benzopyrene (polycyclic aromatic hydrocarbons), phenol, carbazole, diesel fuel and mineral oil (others petroleum products). Minimal medium supplemented with xenobiotics was distributed aseptically in sterile 96-well plates, which were then inoculated with various tested strains, each strain being inoculated at least in 4 repetitions. The inoculated plates were incubated at 37°C for 72 hours. After 72 hours, in the 96-wells plates, optical density was determined, using a multi-mode plate reader PolStar Optima (BMG Labtech, Oretenberg, Germany). From all the isolates tested, strain P32 presented the broadest spectrum of use of the different xenobiotics as the sole source of carbon and energy. The ability of P32 to growth by using various xenobiotic compounds as sole source of carbon and energy is presented in table 3.

Tab. 3. The ability of P32 strain to growth by using various xenobiotic compounds as sole source of carbon and energy.

| Xenobiotic compound | Growth * |
|---|---|
| Ketones and alkanes | |
| Acetone | +++ |
| n-hexane | ++ |
| Aromatic hydrocarbons | |
| Benzene | + |
| Toluene | + |
| Xylene | - |
| Polycyclic aromatic hydrocarbons | |
| Phenanthrene | +++ |
| Naphthalene | ± |
| Benzopyrene | +++ |
| Other petroleum compounds | |
| Carbazol | ++ |
| Phenol | ++ |
| Diesel | +++ |
| Mineral oil | +++ |
| +++ very intense, $DO_{600}$> 1.0; ++ intense, $DO_{600}$> 0.5; + average, $DO_{600}$> 0.1; + weak, $DO_{600}$> 0.05; ± poor, DO > 0,01; - without growth - 0 | |

*Example 3.* It was tested the ability of the isolates to form biofilms *in situ* and *in vitro*. The ability to form biofilms is dependent on group sensitivity (quorum sensing, QS), mediated by signals molecules N-acyl-homoserin-lactone (AHL) (Van Houdt et al., 2007, FEMS Microbiol. Rev. 31:407-424). There were done various tests in order to determine the production of signals QS (AHL) by *P. mexicana* P32 and formation of biofilms on different surfaces.

For the detection of the molecules involved in the group sensitivity (*quorum sensing*) were used two biosensors strains, *Chromobacterium violaceum* CV026 and *A. tumefaciens* NT1.

Mutant CV026 strain was obtained from the wild strain *Chromobacterium violaceum* ATCC 31532, and is used as biosensors for detection of AHL molecules. *Chromobacterium violaceum* CV026 produces purple pigments, called violacein, only when are present in the medium AHL molecules that have the length of the acyl group between $C_4$ and $C_8$ (McClean et al., 1997, Microbiology, 143:3703-3711).

*Agrobacterium tumefaciens* NT1 is a mutant bacterium, obtained from a wild strain, by fusion of *Tra* gene with the *LacZ* gene. The *LacZ* gene is unable to synthesize AHL molecules, and the enzyme β-galactosidase cannot be activated. Hence X-gal present in the medium cannot be cleaved by *Agrobacterium tumefaciens* NT1, and thus in the medium do not form the specific color. If AHL is present (e.g., being synthesized by other bacteria), then a blue-green color appears. The biosensor strain, *A. tumefaciens* NT1, produces pigments of blue-green color only when in the medium are present molecules of N-acyl-homoserin-lactone (AHL), with a the length of the acyl group from $C_4$ and $C_{12}$. (Cha et al., 1998, Mol. Plant-Microbe Interact. 11:1119-1129).

Strain *P. mexicana* P32 and biosensors strains, CV026 and NT1, were refreshed on Luria-Bertani agarized medium (10 g bacto-triptone; 5 g yeast extract, 10 g NaCl, 20 g agar; pH 7.2, sterilized 20 minutes at 121°C) for 24 hours before use. In the case of biosensors strains LB medium was supplemented with chloramphenicol (final concentration of 5 μg/ml) or kanamycin (final concentration of 50 μg/ml).

The test itself consist in streaking biosensors strains, CV026 or NT1, in the center of a Petri dish, and then streaking tested isolate / strain P32 perpendicular to biosensor strains, at a distance of 3 mm. Petri dishes were incubated at 28°C and were analyzed after 24 hours. Formation of a purple color in the growing area of CV026, respectively of the green-blue color in the growing area of NT1, indicated the presence of molecules of acyl-homoserin-lactone produced by tested strain / P32. The test was repeated three times, with both biosensors strains. Each time it was revealed the specific color formation of the different strains of biosensors, which demonstrates the production by the P32 strain of N-acyl-homoserin-lactone (AHL) molecules with a length of the acyl group from $C_4$ and $C_{12}$.

The ability to form biofilms *in vitro* was studied by a spectrophotometric method. Bacteria P32 and others isolates were grown on liquid medium LB at 28°C, till reaching an optical densities $OD_{600} = 2.0$. These cultures were then transferred aseptically on 96-wells plates, 8 repetitions of each strain tested.

The plates were incubated at 25°C for 24 hours. Turbidity was determined as a measure of bacterial growth, using a multi-mode plate reader PolStar Optima (BMG Labtech). After incubation, each well was washed 3 times with sterile water, to remove weakly adherent bacteria to wells walls. The plates were dried at room temperature for 45 minutes and after that each well was treated with a 0.1% Malachite Green solution for 20 minutes. The dye was washed 3 times with sterile water and the formed biofilm was observed as a green color. The quantitative analysis of formed biofilm was done by washing the dye from biofilm with ethyl alcohol, and quantifying the presence of Malachite Green in each well with a reader for micro-plates, at 620 nm. The results obtained for different tested strains are shown in tab. 4, comparing P32 strain with other isolates. It could be notice that strain P32 produces a significant amount of biofilm *in vitro.*

Tab. 4. Absorbance values of Malachite Green dye released from biofilms formed on the walls of the wells of the 96-wells plate.

| Nr | Control | P12 | P16 | P15 | S16 | P32 | P23 | P43 | P60 | P92 | P34 | P52 |
|---|---|---|---|---|---|---|---|---|---|---|---|---|
| 1 | 0.05 | 0.16 | 0.08 | 0.24 | 0.12 | 0.49 | 0.17 | 0.33 | 0.35 | 0.12 | 0.37 | 0.42 |
| 2 | 0.04 | 0.11 | 0.07 | 0.28 | 0.08 | 0.53 | 0.15 | 0.35 | 0.32 | 0.15 | 0.39 | 0.42 |
| 3 | 0.07 | 0.11 | 0.13 | 0727 | 0.09 | 0.56 | 0.11 | 0.29 | 0.33 | 0.14 | 0.38 | 0.37 |
| 4 | 0.05 | 0.11 | 0.09 | 21 | 0.1 | 0.54 | 0.17 | 0.30 | 0.29 | 0.15 | 0.4 | 0.42 |
| 5 | 0.08 | 0.1 | 0.1 | 0.24 | 0.09 | 0.48 | 0.12 | 0.29 | 0.26 | 0.12 | 0.33 | 0.37 |
| 6 | 0.06 | 0.07 | 0.07 | 0.18 | 0.07 | 0.55 | 0.17 | 0.28 | 0.33 | 0.12 | 0.26 | 0.25 |
| 7 | 0.07 | 0.13 | 0.1 | 0.25 | 0.09 | 0.54 | 0.12 | 0.32 | 0.34 | 0.12 | 0.34 | 0.33 |
| 8 | 0.08 | 0.10 | 0.12 | 0.25 | 0.12 | 0.56 | 0.18 | 0.34 | 0.29 | 0.22 | 0.38 | 0.33 |
| average | 0.063 | 0.111 | 0.095 | 0.240 | 0.095 | 0.531 | 0.149 | 0.314 | 0.314 | 0.143 | 0.356 | 0.364 |

For testing *in situ* production of biofilm it was worked by using the method of determination of biofilms formed on sand. In a sterile 24-well plate was added 0.5 ml of sterile sand (fine grains, 0.2 ...0.4 mm). Over the sand was added 0.5 ml of bacterial suspension, prepared as follows. The bacteria were grown on medium LB till reaching a $DO_{600} = 2.0$. The bacteria were centrifuged and re-suspended in the same medium, at the same density. The plates were incubated at 28°C. After 48 hours were collected aseptically 0.1 ml of mixture sand-culture medium, which were brought into a sterile centrifuge tube with known mass. The supernatant formed after centrifugation was removed aseptically. The sand deposited by centrifugation on each of the 24 centrifuge tubes was weighed. Then were added 0.15 ml sterile solution 10 mM $MgSO_4$ in all tubes and vortexed. On the supernatant with re-suspended bacteria the cfu number was assayed by using the dilutions method.

*Pseudoxanthomonas mexicana* P32 strain was compared from the point of view of its capacity to form biofilms with the strain *Serratia plymuthica* HRO-C48 (=DSMZ 12502), recognized for its ability of protecting plant rhizosphere by formation of biofilms (Liu et al., 2007, FEMS Microbiol. Lett. 270: 299-305). From P32 strain were recovered $6,5.10^8 \pm 5, 1.10^7$ cfu $g^{-1}$ of sand, compared to $3.7\ 10^8 \pm 6,3.10^7$ cfu from HRO-C48 strain.

To test the ability of *in situ* biofilms formation on the plant organs it was used the test of re-isolation from the radicle of alfalfa (*Medicago sativa* cv. Magnat) seedlings. The bacteria were grown overnight on liquid medium LB. The suspension was normalized to $2.10^8$ cfu/ml with sterile LB medium, and from this suspension were inoculated 10 $\mu$l per radicle of gnotobiotic alfalfa seedlings (derived from alfalfa seeds, chemically disinfected on the surface and germinated for 5 days under aseptic conditions, in the dark, at 28°C). The inoculation was done on the collet, between radicle and hypocotyl. inoculated seedling were incubated for 24 hours, after that the radicle was aseptically removed from the seedlings and deposited in a sterile centrifuge tube. Over radicle were added 0.15 ml solution 10 mM $MgSO_4$ and vortexed for 5 minutes. The supernatant was recovered by suction and the radicle extracted from the centrifuge tube and dried by aseptic deposition on sterile filter paper. The dried radicle was aseptically lodged on a semisolid medium CM-agarose (10 g/l casaminoacids, 10 g/l of mannitol, 3 g/l agarose), supplemented with 10 ml of sterile nutritive solution. Sterile nutritive solution was obtained by diluting 100 times the stock solution (H metal) and sterilization by filtration. In order to obtain 200 ml of stock solution H metal it was proceeded as will be describe in the following. Were obtained 100 ml of working solution, by diluting 500 times an initial stock solution containing: 1.5 g $CaCl_2.2\ H_2O$, dissolved first; 50 g $MgSO_4.7H_2O$, 0.3 g $MnSO_4.H_2O$, 100 mg $CoCl_2.\ 6\ H_2O$, 100 mg $Na_2MoO_4.\ 2\ H_2O$, 50 mg $CuSO_4.\ 5\ H_2O$, 50 mg $ZnSO_4.\ 7\ H_2O$ and 20 mg $H_3BO_3$. In these 100 ml working solution were added 50 mg $FeSO_4.\ 7\ H_2O$ and 50 mg of ascorbic acid.

From the radicle were obtained dendritic colonies, with a specific aspect which demonstrates the existence of mobility characteristics associated with the formation of biofilms on the underground parts of cultivated plants.

In conclusion the strain of *Pseudoxanthomonas mexicana* P32 forms biofilms *in vitro* and *in situ,* both on mineral particles and on plant roots.

*Example 4.* It was tested the ability of strain P32: to produce volatile compounds, with an activity of stimulating plant growth, to solubilize phosphorus from its insoluble forms, organic and inorganic, and to stimulate, under controlled conditions, alfalfa seeds germination and alfalfa seedlings growth and development,. Production of volatile compounds involved in plant stimulation was demonstrated through assaying the production of acetoin. Acetoin (3-hydroxy-2-butanone, HB) is a physiologically important metabolite, excreted by micro-organisms when they are grown in a culture medium containing glucose or other degradable carbon source through the Embden-Meyerhof pathway. The formation of acetoin is revealed by Voges-Proskauer reaction, and serves as a marker in microbial classification, having an important role in regulating the rate of NAD/NADH and in carbon storage. Production of acetoin demonstrates the ability of a given strain to produce 2,3-butandiol, and of volatile compounds involved in the stimulation of plants growth (Ryu et al., 2003. Proc. Natl. Acad. Sci. USA 100:4927-4932), Strain P32 was grown for 24 hours in LB medium (incubated at 28°C with agitation), then 100 $\mu$l culture were inoculate in 5 ml of liquid medium with glucose (proteose-peptone 7 g, glucose 5 g, NaCl 5 g, distilled water to 1000 ml), distributed in test tubes, sterilized for 15 minutes at 1 atm. The culture was prepared in 3 repetitions and tested for production of acetoin, 3, 5 and 7 days after inoculation and incubation at 28°C. A 10% NaOH solution, 1 ml distributed in each test tube, followed by the addition in each test tube of 1 mg of creatine, was used as reagent, and the mixture was shaken vigorously. The red color appeared, after 30-60 minutes at room temperature, indicating the presence of acetyl - methyl - carbinol, thus a positive reaction, which shows the ability of P32 bacteria to produce volatile compounds that stimulate plant growth.

In another series of experiments the ability of P32 bacteria to solubilize phosphorus from its insoluble compounds was tested. Pikovskaya agarized medium, which contains (per liter): 0.5 g yeast extract, 10 glucose, 5 g $Ca_3(PO_4)_2$, 0.5 g $(NH_4)_2SO_4$, 0.2 g KCl, 0.1 g $MgSO_4.\ 7\ H_2O$, 0.0001 g $MnSO_4.H_2O$, 0.0001 g $FeSO_4.\ 7\ H_2O$ and 15 g agar, was used to test the ability to solubilize mineral phosphorus. In this medium the P32 bacterial strain produce a halo around colonies, thus they solubilized the mineral phosphorus.

The capacity to solubilize organic phosphorus was tested on PSM (*phytate screening medium*), which contains (per liter): 10 g glucose, 4 g sodium phytate, 2 g $CaCl_2$, 5 g $NH_4NO_3$, 0.5 g KCl, 0.5 g $MgSO_4.\ 7H_2O$, 0.01 g $FeSO_4.\ 7\ H_2O$ 0.01 g $MnSO_4 \cdot H_2O$, 15 g agar. Also on this medium P32 bacterial strain produced a halo around the colonies, so they solubilize the organic phosphorus from phytates.

The activity of plant growth stimulation of P32 strain was revealed by experiments performed in controlled conditions, using alfalfa seedlings, *Medicago sativa*, cv. Magnat. For inoculation, alfalfa seeds were disinfected in two stages. The first disinfection was carried out in 70% ethanol, for 30 seconds, with agitation at 60 rpm. After the removal of ethanol, seeds were rinsed three times with sterile distilled water.

The second disinfection was done with a solution of sodium hypochlorite 5%, for 15 minutes. Subsequently, were made rinses with sterile distilled water, for 25 minutes periods, repeated for two hours. The disinfected seeds were stored at 4°C, in the dark, on the moisten filter paper.

P32 strain was cultivated on LB liquid medium, at 28°C and 150 rpm, for 16 hours. Seeds inoculation was done at a bacterial concentrations of $10^6$ cfu/ml. Seed bacterization was done by dipping the seeds into the bacterial suspensions for 15 minutes, at room temperature and on an agitation speed of 20 rpm. The control was made using alfalfa seeds, sterilized as above, which were immersed in sterile distilled water in the same conditions.

Alfalfa seeds inoculated according to the method described above were germinated in Petri plates (diameter of 9.4 cm), with 0.8% agarized water, in number of 20 seeds/plate, 5 repetitions. Each treatment consisted of 100 seeds. Incubation temperature was 28°C. Observations were done regarding the number of germinated seeds at 24, 48 and 72 hours.

To determine the influence of P32 strain on the development of alfalfa seedlings, alfalfa seeds, inoculated with bacterial suspension in concentration of $10^6$ or $10^8$ cfu/ml, were distributed in Petri dishes (with agarized water, diameter of 9.4 cm), 10 seeds/plate, 3 repetitions (30 seeds per treatment). Over the agarized water were added 5 ml of mineral medium for plants with the following composition: 0.4 g $NH_3H_2PO_4$; 2.4 g $KNO_3$; 1.6 g $(NO_3)_2$; 0.8 g $MgSO_4$, agarized with 2 grams per liter. The plates were incubated in the dark at 28°C. Observations were done regarding the stimulating effect on the growth of the radicles and hypocotyls of alfalfa seedlings after 72 hours, determining the length of the radicles and height of the hypocotyls.

Determinations regarding the influence of P32 strain on the germination and development of alfalfa seedlings were compared against a control group treated with sterile distilled water. The results, presented in tab. 5 and 6, sustain the existence of a phytostimulatory effect of P32 bacteria on alfalfa seedlings, and particularly on the root system.

Tab. 5. Influence of bacterial inoculum with P32 strain on the germination capacity of alfalfa seeds (*Medicago sativa* cv. Magnat).

| Bacterial strain Treatment | Inoculum concentrations (cfu/ml) | % of the seeds germinated after | | |
|---|---|---|---|---|
| | | 24 h | 48 h | 72 h |
| Control, sterile distilled water | - | 72 | 86 | 90 |
| P32, *Pseudoxanthomonas mexicana* | $10^6$ | 84 | 90 | 98 |

Tab. 6. Testing of the stimulating effect on the growth of alfalfa seedlings (*Medicago sativa* cv. Magnat) of P32 strain (72 hours after inoculation)

| Bacterial strain | Inoculum concentrations (cfu/ml) | Seed Germination | | Length radicle (cm) | Hypocotyls height (cm) |
|---|---|---|---|---|---|
| | | of 30 seeds | % | | |
| P32 | $10^6$ | 29 | 96.7 | 3.35 | 2.32 |
| P32 | $10^8$ | 29 | 96.7 | 3.87 | 2,43 |
| Control, not-inoculated | - | 25 | 83.3 | 2.68 | 2.24 |
| | | | DL 5% | 0.38 | 0.27 |

In conclusion the P32 strain produce volatile compounds which are involved in the stimulation of plant growth, solubilize phosphorus from its insoluble forms, organic and inorganic, and stimulate germination of alfalfa seeds and alfalfa seedlings growth, under controlled conditions.

*Example 5.* It was tested *in vitro* antagonism towards phytopathogenic fungi (*Rhizoctonia solani, Fusarium graminearum, Pythium ultimum, Phytophthora megasperma* f. sp. *medicaginis, Sclerotinia sclerotiorum, Aphanomyces euteiches, Botrytis cinerea*) of the P32 strain, and its ability to protect *in vivo* alfalfa plants against phytopathogenic fungi (*Rhizoctonia solani, Fusarium graminearum, Pythium ultimum, Phytophthora megasperma f. sp. medicaginis, Aphanomyces euteiches*) that produce seedlings damping-off.

*In vitro* testing of the antagonistic activity of P32 strain was carried out on: potato dextrose agar medium (PDA, Scharlau, Barcelona, Spain) toward *Rhizoctonia solanii* ATCC 66873, *Fusarium graminearum* DSM4527, *Pythium ultimum* DSM 62987, *Sclerotinia sclerotiorum* DSM 1946, *Botrytis cinerea* DSM 5144; on agarized V8 (V8 juice 50 ml, 0.2 g CaCO3, 20 g agar, pH 6.5) against *Phytophthora megasperma* f. sp. *medicaginis* ATCC 42154; and agarized medium with corn extract (cormmeal agar, Oxoid Thermo Scientific, Hampshire, United Kingdom) towards *Aphanomyces euteiches* ATCC 08988. Strain P32 (from a 24 hours culture) was inoculated on agarized media by streaking an straight line with a loop, at a distance of 3 cm from a calibrated disc of mycelium (5 mm), of each of the seven phytopathogenic fungi studied. Petri plates inoculated in this manner were incubated at 28°C and analyzed in terms of inhibition zone (mm) at 24, 48 and 72 hours. The experience was repeated five times. The results, presented in table 7 from bellow, shown that P32 strain produces metabolites with antifungal activity, which inhibited the development of phytopathogenic fungi used into this experiment. The largest area of inhibition was registered against *Botrytis cinerea* (7 mm). Biological action is significant also towards *R. solani, P. ultimum, Phytophthora megasperma* f. sp. *medicaginis, S. sclerotiorum, A. euteiches* (5 mm). The bacteria P32 have a moderate antagonism against *F. graminearum* (2.5 mm).

Tab. 7. *In vitro* testing of the antagonistic activity of the strain *Pseudoxanthomonas mexicana* P32 on the mycelia growth of phytopathogenic fungi (zone of inhibition after 72 h, mm)*.

| Phytopathogen fungus | Zone of inhibition (mm) induced by the strain P32 |
|---|---|
| *Rhizoctonia solani* ATCC 66873 | 5 |
| *Fusarium graminearum* DSM4527 | 2.5 |
| *Pythium ultimum* DSM 62987 | 5 |
| *Phytophthora megasperma* f.sp. *medicaginis* ATCC 42154 | 5 |
| *Sclerotinia sclerotiorum* DSM 1946 | 5 |
| *Botrytis cinerea* DSM 5144 | 7 |
| *Aphanomyces euteiches* ATCC 08988 | 5 |
| * average of 5 measurements | |

P32 strain was tested, under controlled conditions, regarding its efficacy in controlling phytopathogenic fungi from soil (*Rhizoctonia solani, Fusarium graminearum, Pythium ultimum, Phytophthora megasperma* f. sp. *medicaginis, Aphanomyces euteiches*), which produce seedlings damping-off. To perform this test, were used seeds of alfalfa (*Medicago sativa*) cv. Magnat. Seeds, previously disinfected with 4% hypochlorite solution, were submerged in a mixture consisting of bacterial cell suspension with the concentration of 1 x $10^9$ cfu/ml and 1% methylcellulose in phosphate buffer (w/v). Phytopathogenic fungi were grown on the Potato-Dextrose-Agar medium (PDA, Scharlau) - *Rhizoctonia solanii* ATCC 66873, *Fusarium graminearum* DSM4527, *Pythium ultimum* DSM 62987, *Sclerotinia sclerotiorum* DSM 1946, *Botrytis cinerea* DSM 5144, agarized V8 (V8 juice 50 ml, 0.2 g CaCO3, 20 g agar, pH 6.5) - *Phytophthora megasperma* f.sp. *medicaginis* ATCC 42154, medium with corn extract (cormmeal agar, Oxoid) - *Aphanomyces euteiches* ATCC 08988, in order to obtain a uniform growth of the mycelium. After 4-5 days of incubation at 28°C, the mycelium was shredded and used for inoculation of 200 ml of liquid similar medium (potato glucose for *R. solani, F. graminearum, P. ultimum, S. sclerotiorum, B. cinerea,* medium V8 liquid for *P. megasperma* f. sp. *medicaginis* and liquid corn extract medium for *A. euteiches*) distributed in erlenmeyer flasks. Inoculated erlenmayer flasks were incubated for 4 to 5 days at 28°C. The formed spores were separated from the mycelium by filtration using a sterile cloth. Bacterized alfalfa seeds were sown in sterile plastic bags (cyg - Mega International, West St. Paul, MN, USA) and placed in a plant growing chamber, environmental Fitotron® chamber (Model GSC 120 LED, Weiss Gallenkamp, Lougborough, United Kingdom), where they were kept on 160 μmol photons/m$^2$/s, with 12 hours photoperiod, at 21 °C and 70% humidity. Each bag was moistened with 2 ml of Hoagland nutritive solution, infected with spores in the concentration of $10^6$ spores/liter of solution. In uninfected control for moistening was used only Hoagland nutrient solution. The seeds of the chemical control were treated with Tachigaren 70 PU (Sumi-Agro Romania, Bucharest, Romania, himexazol 70%, the equivalent dose of 6 g/kg). The bags were moistened each 2 days during 4 weeks, using for re-moistening sterile nutritive Hoagland solutions.

After 4 weeks, the plants have been analyzed in terms of the characteristic symptoms of each disease, especially on browning of collet and of the upper part of the radicles. Efficacy was calculated using the Abbot formula:

$$Efficacy\ \% = (1 - nPS_t / nPS_i) * 100\ (1)$$

Where:

nPS$_i$ is the number of healthy plants on the un-infected control;
nPS$_t$ is the number of healthy plants in the treatment.

The data were statistically processed by analysis of variance (Statistica, StatSoft, Tulsa, OK, USA). The efficacy of the treatment with strain *P. mexicana* P32 in controlling the damping-off symptoms of alfalfa seedlings is presented in table 8 from bellow.

Tab. 8. The efficacy of the treatment with strain *P. mexicana* P32 in controlling the damping-off symptoms of alfalfa *(Medicago sativa* cv. Madrigal) seedlings.

| Treatment | % healthy germinated plants | Efficacy (%) | % healthy germinated plants | Efficacy (%) | % healthy germinated plants | Efficacy (%) |
|---|---|---|---|---|---|---|
| *Fungal inoculum* | *R.F.P.* * | | *P. m. m.* | | A. e. * | |
| Untreated, infected | 45 | - | 34 | - | 53 | |
| Tachigaren 70 PU (4 g/kg) | 93 | 87,27 | 98 | 96,97 | 94 | 87,23 |
| *P32* | *90* | *81,82* | *98* | *93.44* | *89* | *80,85* |
| * R.F.P- *Rhizoctonia solani, Fusarium graminearum, Pythium ultimum*; P.m.m.- *Phytophthora megasperma f.sp. medicaginis*; A.e., *Aphanomyces euteiches* | | | | | | |

In the given experimental conditions the efficacy of strain P32 in protecting alfalfa plants against damping-off disease is comparable to that of the chemical used as standard, being more than 80% in all situations.

In conclusion the tested strain presents a significant protection action on the alfalfa plants against phytopathogenic fungi from the soil.

*Example 6.* It was tested the innocuity of P32 strain using *Galleria mellonella* test, one of the model organisms proposed for the determination of pathogenicity of proteobacteria (Seed and Dennis, 2008, Infect. Immune. 76:1267-1275). Larvae have been reared on a rearing substrate, at 30°C, the substrate containing: cornmeal 400 g, wheat flour 200 g, wheat bran 200 g. All these components have been kept in the freezer for 7-10 days and after that these were sterilized by maintaining at 70°C for 2 hours. To these sterilized components were added 100 g ground dry yeast and 200 g powdered milk. To this powdery mixture were added 700 ml of liquid mixture, composed of 350 ml honey and 350 ml glycerol with suspended bees wax (1:1 w/v). The final composition was homogenized in a mixer and was kept in a plastic box, at a temperature of 4°C.

The larvae of the third age were kept at 4°C above the rearing substrate mentioned above. For infection, it was used a Hamilton syringe of 5 $\mu$l. 5 $\mu$l of bacterial suspension were injected through the left side of the last segment. After the injection, the larvae were placed in the incubator, in the dark, at 30°C, the optimal temperature for their growth and development. Serial dilutions were made, combining bacterial concentration between $10^6$ and 0, in solution 10 mM MgSO$_4$, which were used for injection on larvae of *G. mellonella.* On the control treatment, larvae were injected with 5 $\mu$l 10 mM solution MgSO$_4$ + 1.2 mg/ml ampicillin, to assess any potentially lethal effect of the injection procedure. From each dilution were infected 20 larvae, being done three repetitions / dilution. Monitoring of larvae (dead, alive, chrysalides) was done at 24 ... 72 hours after infection, at 30°C (tab. 9).

Tab. 9. Mortality of larvae of *Galleria mellonella* injected with suspension $10^8$ cfu/ml of the test strain.

| Treatment | Repetition | 24 hours after infection | | 48 hours after infection | | 72 hours after infection | | |
|---|---|---|---|---|---|---|---|---|
| | | alive | death | alive | death | alive | death | chrysalides |
| Control 10 mM MgSO$_4$ + 1.2 mg/ml ampicillin (DO 0,000) | R1 | 20 | 0 | 20 | 0 | 20 | 0 | 2 |
| | R2 | 20 | 0 | 20 | 0 | 19 | 1 | 1 |
| | R3 | 20 | 0 | 19 | 0 | 18 | 1 | - |
| P32 $10^6$ cfu/ml | R1 | 20 | 0 | 19 | 1 | 18 | 2 | - |
| | R2 | 18 | 2 | 18 | 2 | 18 | 2 | - |
| | R3 | 20 | 0 | 20 | 0 | 19 | 1 | 1 |

The data from tab. 9 demonstrates that strain tested, P32, practically do not presents pathogenicity against larvae of *Galleria mellonella*, having no ability to multiply in the larvae body and to produce bacteremia, although were injected in high concentrations into the larvae lymph fluid. The strain P32 is not pathogenic for metazoa organisms and do not present a risk for human health.

*Example 7.* Strain *P. mexicana* P32 was multiplied and included in a composition which would promote its colonization of the soil and/or rhizosphere. Multiplication was done on an industrial medium OK (patent RO 118717), containing 10 g/l glycerol, 1.5 g/l autolysed yeast, 1.0 g/l K$_2$HPO$_4$, 0.2 g/l MgSO$_4$. 7H$_2$O, 0.1 g/l CaCl$_2$.2 H$_2$O. pH of the liquid medium was adjusted to pH 6,5 with 1 N NaOH, medium was distribute as 150 ml to 1 l erlenmeyers flasks, sterilized, inoculated and agitated at 150 rpm on an orbital shaker (Unimax 1010, The Heidolph Instruments) at 28°C, 48 hours. After 48 hours it was reached a level of the bacterial population of 5.2x$10^9$ cfu/ml. This bacterial suspension was used for formulation as granules. 35 ml of suspension were added over 90 g of mixture consisting of 76 g oatmeal flour, 10 g mixture which includes ethyl esters of fatty acids, lecithin, potassium soaps, glycerol, lipids of rapeseed oil, and 4 g polyvinyl alcohol (26-88, powder, EMPROVE ®, Merck, Darmstadt, Germany). The resulting paste was extruded on a pasta machine (Model TR95A, Helco, Craiova, Romania), and the resulting noodles were granulated on a spheronization equipment (model Spheronis-250 m Grabler, Ettlingen, Germany). The granules were dried in a dryer fluid bed (TC20 Model, Retsch, Germany), at a maximum temperature of 40°C.

The mixture of ethyl esters of fatty acids, lecithin, potassium soap, glycerin, unsaponifiable lipids from rapeseed oil, was obtained according to the procedure described below. 1 000 g of degummed rapeseed oil, with the characteristics shown in table 10, was brought-up in a 2 liters stainless steel autoclave, with stirring and heating, under protective atmosphere of nitrogen.

Tab. 10. Characteristics of degummed rapeseed oil used

| Water and volatile compounds | % m/m | 0.4 |
|---|---|---|
| Unsaponifiable substances | % m/m | 1.4 |
| Free fatty acids | % m/m | 1.9 |
| Saponification number | mg KOH/g | 169.5 |
| The average fatty acid composition (% w/w): C16: 2.4; C18: 2; C18-1: 10.0; C18-2: 24.5; C18-3: 7.3; C20-1: 4.5; C22-1: 42.4 | | |

25 g of potassium hydroxide purity 98% were dissolved on 105 g of ethanol with 0.3% water, and the resulting solution was added in autoclaves over degummed rapeseed oil. It was started the agitation and the heating to 40°C. After a reaction time of 8 hours, the reaction was stopped, and the reaction mass was cooled till reaching room temperature. 1125 g transparent reaction mass (R1) were collected. 500 g of R1 were treated with technical oleic acid, yielding a product (P1) having the following composition: (% w/w): fatty acid ethyl esters (FAEE) 74.5; not - saponified lipids 5.9; glycerol 7.1; potassium soap 11.4 and water 1.1. 131 g of the product of the reaction (P1) was treated under vigorous shaking with 69 g soy lecithin liquid emulsifier, modified, with a hydrophilic - lipophilic balance, HLB, higher than 8 (Thermolec® WFC, Archer Daniels Midland, Decatur, IL, USA), resulting a mixture with the following composition, (% w/w): fatty acid ethyl esters (FAEE) 48.7; not reacted / unsaponifiable lipids from rapeseed oil 4; glycerol 4.5; potassium soap 7.5, lecithin 34.6 and water 0.7. This mixture was the one from which were taken 10 g, which were added over 76 g oatmeal flour and 4 g polyvinyl alcohol, and used for the preparation of controlled-release composition containing strain P32.

Controlled-release formulation based on strain *P. mexicana* P32 obtained on this manner consist of 76 parts oatmeal flour, 4.8 parts ethyl esters of fatty acids, 4 parts polyvinyl alcohol, 3.5 parts lecithin, 0.75 parts potassium soaps, 0.45 parts glycerol, 0.4 parts fats from rapeseed oil, the rest up to 100 parts water, and min. $10^8$ cfu/g *P. mexicana* P32.

The number of P32 bacteria in the obtained composition was verified using WST-1 indicator, in accordance with the method described by Johnsen et al., 2002, Appl. Environ. Microbiol. 68:2683-2702.

*Example 8.* P32 strain and the controlled release composition done according to Ex. 7 were tested regarding the capacity of degradation of benzopyrene, contaminant in the soil, using an experiment of microcosm type. Soil used, a cambic chernozem from Fundulea, was previously analyzed in terms of presence of aromatic hydrocarbons, being found as free of aromatic hydrocarbons. The used soil had a water retention capacity (WHC) of 65% and the organic matter content of 3%. The soil was dried, sieved through a 1.18 mm sieve and then distributed, as 6 g, in amber glass bottles of 50 ml. The soil form each bottle was homogenized with a benzopyrene (Sigma-Aldrich) solution, obtained by solving benzopyrene in acetone, in order to achieve a final concentration of 300 mg/kg soil.

A total of 48 bottles of soil supplemented with benzopyrene were used in an experiment, randomized in a Latin square arrangement, with four treatments, each of these treatments with four repetitions, each repetition represented by 3 bottles. The four experimental treatments tested were:

$V_1$ - untreated control
$V_2$ - soil treated with 1 g/kg of integral barley flour;
$V_3$ - soil treated with 1 ml suspension $10^8$ cfu/ml P32 strain per kg;
$V_4$ - soil treated with 1 g/kg of composition in accordance with Ex. 7

Bottles were incubated at room temperature for 4 weeks, with water added weekly to keep the WHC at 65%. At the end of 4 weeks it was determined the number of bacteria that degrade polycyclic aromatic hydrocarbons using WST-1 indicator, in accordance with the method described by Johnsen et al., 2002, Appl. Environ. Microbiol. 68:2683-2702, and the quantity of benzopyrene. For the determination of benzopyrene, 2 g of soil were extracted with 4 ml of n-hexane (Merck, Darmstadt, Germany), mixed with 1.5 ml of 15% Triton X-100 (Sigma-Aldrich). After shaking at 200 rpm for 24 hours, the samples were stored at -20°C for another 24 hours. Then the hexane layer was taken-out with a Pasteur pipette, dried over $Na_2SO_4$, anhydrous, (Merck), and filtered through a PTFE filter. The concentration of benzopyrene was determined by gas chromatography, on a gas chromatograph Perkin Elmer Clarus 500 (Perkin Elmer, Waltham, MA, USA), with a flame ionization detector, after separation on a capillary column, 30 m×0.32 mm internal diameter and ×0.25 μm layer thickness of 5% phenyl-methyl-siloxan. The percentage of remaining benzopyrene was calculated with the following formula:

$$100 - (C_M - C_T) / C_M \times 100 \quad (2)$$

where:

$C_M$ is the concentration in untreated control;
$C_T$ is the concentration in a given treatment.

The data were processed by analysis of variance (Statistica, StatSoft). The results are shown in tab. 11 from below. These results support the efficacy of P32 strain for bioremediation of soil contaminated with benzopyrene.

Tab. 11. The influence of strain and composition P32 based on the degradation of benzopyrene from soil in experiment of microcosm.

| Experimental version | The percentage of the remaining benzopyrene* | Number of bacteria that degrade polycyclic aromatic hydrocarbons (log cfu/g dried soil) |
|---|---|---|
| $V_1$ -untreated control | 93.55 ± 4.85 | 0 |
| $V_2$ -soil treated with 1 g/kg of integral barley flour | 77.47 ± 9.82 | 6.15 ± 0.68 |
| $V_3$ -soil treated with 1 ml suspension $10^8$ cfu/ml P32 strain per kg | 34.15 ± 7.54 | 7.85 ± 0.22 |

(continued)

| Experimental version | The percentage of the remaining benzopyrene* | Number of bacteria that degrade polycyclic aromatic hydrocarbons (log cfu/g dried soil) |
|---|---|---|
| $V_4$ -soil treated with 1 g/kg of composition in accordance with Ex. 7 | $5.2 \pm 2.3$ | $9.66 \pm 0.27$ |
| * the initial concentration was 300 mg/kg soil | | |

The inclusion of P32 strain in the composition obtained in accordance with the invention increase its ability to colonize soil and to degrade polycyclic aromatic hydrocarbons.

*Example 9*. Strain P32 and the controlled release composition done according to Ex. 7 were tested for the capacity to support phytorhizoremediation of a soil contaminated with total petroleum hydrocarbons. Soil used, a cambic chernozem from Fundulea, with characteristics similar to those described in Ex. 8, was contaminated with petroleum originating from an oil wells near Câmpina (Petrom, Romania, Bucharest). 40 g of petroleum were mixed with 2 kg of soil, the mixture being stirred for 2 min in a hermetically sealed plastic container. After mixing, the soil contaminated with petroleum was transferred into plastic pots, 15 cm in diameter, and was sown with seeds of alfalfa (*Medicago sativa*, cv. Magnat). Also were kept some pots with contaminated soil not-sown with alfalfa seeds.

A number of 80 pots were used to carry out an experiment with five treatments, each of these with four repetitions, each repetition consisting of 4 pots. The five experimental treatments tested were:

$V_1$ -control, soil untreated and not sown with alfalfa;
$V_2$ - soil sown with alfalfa;
$V_3$ - soil sown with alfalfa, treated with equiv. 100 g/m$^2$ integral barley meal;
$V_4$ - soil sown with alfalfa, treated with equiv. 100 ml suspension $10^8$ cfu/ml strain P32 per m$^2$;
$V_5$ - soil sown with alfalfa, treated with equiv. 100 g/m$^2$ of composition in accordance with Ex. 7.

Pots were kept in greenhouse conditions, at $26 \pm 3°C$, with a 16 hours photoperiod, supplemented with a light intensity of 160 $\mu$E/m$^2$/s, generated from halide lamps, when light intensity decreased below 500 $\mu$E/m$^2$/s. Plants were grown for six months, at a relative humidity ranging between 55 and 85%. After six months the experiment was terminated, the roots of alfalfa plants were taken-out, cleaned from soil and then weighted. Samples of 5 g soil were used for the determination of the total hydrocarbons by adapted EPA method 418.1. Soil sample, weighed exactly, was introduced into an extraction cartridge, and extracted with 100 ml of methylene chloride, for ten cycles, in a Soxhlet apparatus. For IR analysis, an exactly measured volume of methylene chloride extracting solution, between 1.5 ...2 ml, was transferred to an evaporation vial, and dried under a stream of nitrogen in a niche. The residue was quantitatively resumed in 10 ml of dichlorodifluoromethane (Fisher Scientific, Schwerte, Germany); it was added 1 g silica gel (Sigma-Aldrich) for the removal of polar organic compounds. The content of total petroleum hydrocarbons (THP) was determined using an IR total hydrocarbon analyzer (Model HC-404, Buck Scientific, East Norwalk, CT, USA). Absorbance values were transformed into concentrations based on a standard plot, made with dilutions from 10 mg/kg and 1000mg/kg, from a reference material (HC-404, Buck Scientific reference standard).

In soil samples was determined the number of bacteria that degrade polycyclic aromatic hydrocarbons using WST-1 indicator, in accordance with the method described by Johnsen et al., 2002, Appl. Environ. Microbiol. 68:2683-2702

The data were processed by analysis of variance (Statistica 10). The results are presented in tab. 12 from below. These results support the efficacy of P32 strain, and of composition based on it, in association with alfalfa plants, for phytorhizoremediation of soil contaminated with total petroleum hydrocarbons.

Tab. 12. Influence of P32 strain and of composition based on it, in association with alfalfa plants, for phytorhizoremediation of soil contaminated with petroleum hydrocarbons, in greenhouse conditions.

| Experimental version | Average concentration THP * (mg/kg) | Number of bacteria that degrade polycyclic aromatic hydrocarbons (log cfu/g dry soil) | The average mass of alfalfa plants/plant |
|---|---|---|---|
| $V_1$ - witness the ground untreated | 9450a | $4.24 \pm 54$ c | - |
| $V_2$ -soil seeded with alfalfa | 8270b | $4.65 \pm 0, 86c$ | 9, 5 d |

(continued)

| Experimental version | Average concentration THP * (mg/kg) | Number of bacteria that degrade polycyclic aromatic hydrocarbons (log cfu/g dry soil) | The average mass of alfalfa plants/plant |
|---|---|---|---|
| $V_3$ -seeded with alfalfa soil treated with equiv. 100 g/m$^2$ integral barley flour | 7420c | $5.05 \pm 78bc$ | 10, 1 c |
| $V_4$-soil seeded with alfalfa treated with equiv. 100 ml suspension $10^8$ cfu/ml strain P32 per m$^2$ | 6850d | $5.45 \pm 0, 37b$ | 10, 7b |
| $V_5$ - soil seeded with alfalfa treated with equiv. 100 g/m$^2$ in accordance with e.g. 7 composition. | 6170e | $6.39 + 0, 42a$ | 11, 2nd |
| * Values followed by the same letter does not differ significantly for $P > 0.05$; the initial concentration was of 20 g of total petroleum hydrocarbons (THP)/kg soil | | | |

The inclusion of P32 strain in the composition done in accordance with the invention increase its capacity to colonize the rhizosphere of alfalfa plants and for phytorhizoremediation together with these of soil contaminated with total petroleum hydrocarbons.

## Claims

1. Strain of *Pseudoxanthomonas mexicana* P32 NCAIM (P) B 001414.

2. Controlled-release composition based on strain *P. mexicana* P32 of claim 1, **characterized in that** it consists of 76 parts oatmeal flour, 4.8 parts ethyl esters of fatty acids, 4 parts polyvinyl alcohol, 3.5 parts lecithin, 0.75 parts potassium soap, 0.45 parts glycerol, 0.4 parts of fats from rapeseed oil, the rest till 100 parts water, and min. $10^8$ cfu/g *P. mexicana* P32.

## Patentansprüche

1. Stamm von *Pseudoxanthomonas mexicana* P32 NCAIM (P) B 001414.

2. Zusammensetzung mit kontrollierter Freisetzung basiert auf der *P. mexicana* Stamm gemäß Anspruch 1, **dadurch gekennzeichnet, dass** sie aus 76 Teilen Hafermehl, 4,8 Teilen Ethylestern von Fettsäuren, 4 Teilen Polyvinylalkohol, 3,5 Teilen de Lecithin, 0,75 Teil Kaliumseife, 0,45 Teil de Glycerin, 0,4 Teil Rapsöl Fett, der Rest bis 100 Teile Wasser, und mindestens $10^8$ cfu/g *P. mexicana* P32 besteht.

## Revendications

1. Souche de *Pseudoxanthomonas mexicana* P32 NCAIM (P) B 001414.

2. Composition à libération contrôlée basée sur la souche *P. mexicana* P32 selon la revendication 1, **caractérisée en ce qu'**elle est composée de 76 parties de farine d'avoine, 4,8 parties d'esters éthyliques d'acides gras, 4 parties d'alcool polyvinylique, 3,5 parties de lécithine, 0,75 parties de savon de potassium, 0,45 parties de glycérine, 0,4 parties de graisses d'huile de colza, le solde pour faire 100 parties étant de l'eau, et au moins $10^8$ cfu/g de *P. mexicana* P32.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5543317 A
- KR 20030066948
- CN 102250788
- JP 3618785 B
- RU 2406758
- RO 118717

### Non-patent literature cited in the description

- **MAHENTHIRALINGAM et al.** *J. Appl. Microbiol.,* 2008, vol. 104, 1539-1551
- **RYAN et al.** *Nat. Rev. Microbiol,* vol. 7, 514-525
- **GOVAN ; DERETIC.** *Microbiol. Rev.,* 1996, vol. 60, 539-574
- **LOONEY.** *Lancet Infect. Dis,* 2009, vol. 9, 312-323
- **KUIPER et al.** *Mol. Plant Microbe Interact.,* 2004, vol. 17, 6-15
- **LUGTENBERG ; KAMILOVA.** *Annu. Rev. Microbiol.,* 2009, vol. 63, 541-556
- **SASSER.** Methods in Phytobacteriology. Akademiai Kiado, 1990, 119-204
- **ROSE et al.** *Int. J. System. Evol. Microbiol.,* 2004, vol. 54, 2245-2255
- **VAN HOUDT et al.** *FEMS Microbiol. Rev.,* 2007, vol. 31, 407-424
- **MCCLEAN et al.** *Microbiology,* 1997, vol. 143, 3703-3711
- **CHA et al.** *Mol. Plant-Microbe Interact.,* 1998, vol. 11, 1119-1129
- **LIU et al.** *FEMS Microbiol. Lett.,* 2007, vol. 270, 299-305
- **RYU et al.** *Proc. Natl. Acad. Sci. USA,* 2003, vol. 100, 4927-4932
- **SEED ; DENNIS.** *Infect. Immune.,* 2008, vol. 76, 1267-1275
- **JOHNSEN et al.** *Appl. Environ. Microbiol.,* 2002, vol. 68, 2683-2702